# EUROPÄISCHE PATENTANMELDUNG

(11) **EP 4 574 012 A1**
(43) Veröffentlichungstag der Anmeldung: **25.06.2025**
(21) Anmeldenummer: 24220800.7
(22) Anmeldetag: 17.12.2024
(51) Int. Cl.: A61B 1/00, A61B 1/04, A61B 1/06

(54) **BILDGEBUNGSVORRICHTUNG, INSBESONDERE ENDOSKOPISCHE, EXOSKOPISCHE UND/ODER MIKROSKOPISCHE BILDGEBUNGSVORRICHTUNG**

(30) Priorität: 18.12.2023 DE 102023135636
(71) Anmelder: Karl Storz SE & Co. KG, 78532 Tuttlingen (DE)
(72) Erfinder: Buschle, Lukas, 78532 Tuttlingen (DE); Röschert, Isabelle, 78532 Tuttlingen (DE)

(57) **Zusammenfassung**

Die Erfindung betrifft eine Bildgebungsvorrichtung (10), insbesondere eine endoskopische, exoskopische und/oder mikroskopische Bildgebungsvorrichtung, umfassend: eine Beleuchtungsvorrichtung (12), welche zur Beleuchtung eines Untersuchungsbereichs (14) vorgesehen ist, eine Bildaufnahmeeinheit (20), welche eine Hyperspektralsensorik (66) zur Aufnahme zumindest eines hyperspektralen Abbilds des Untersuchungsbereichs (14) aufweist, und eine Analyseeinheit (22) zur Analyse des Abbilds, wobei die Beleuchtungsvorrichtung (12) zur gleichzeitigen Anregung eines ersten Lumineszenzfarbstoffs (16) und zumindest eines zweiten Lumineszenzfarbstoffs (18) vorgesehen ist.

## Beschreibung

Die vorliegende Erfindung betrifft ein Bildgebungsvorrichtung, insbesondere eine endoskopische, exoskopische und/oder mikroskopische Bildgebungsvorrichtung, sowie ein Verfahren zum Betrieb einer Bildgebungsvorrichtung.

Aus dem Stand der Technik sind Bildgebungsvorrichtungen wie beispielsweise endoskopische oder exoskopische Vorrichtungen bekannt, die Multispektral- oder Hyperspektralbilder erzeugen. Multispektral- oder Hyperspektralbilder weisen neben zwei räumlichen Dimensionen, wie sie etwa ein herkömmliches Bild einer Kamera hat, eine spektrale Dimension auf. Die spektrale Dimension umfasst mehrere Spektralbänder (Wellenlängenbänder). Multispektrale und hyperspektrale Bilder unterscheiden sich im Wesentlichen in der Anzahl an und der Breite von ihren spektralen Bändern.

Es sind einige Bildgebungsvorrichtungen zur Erzeugung solcher Multispektral- oder Hyperspektralbilder, insbesondere im Kontext medizinischer Anwendungen, bekannt. In DE 20 2014 010 558 U1 ist beispielsweise eine Vorrichtung zur Aufnahme eines Hyperspektralbilds eines Untersuchungsgebietes eines Körpers beschrieben. In der Vorrichtung sind ein Eingangsobjektiv zur Erzeugung eines Bilds in einer Bildebene sowie eine schlitzförmige Blende in der Bildebene zur Ausblendung eines schlitzförmigen Bereichs des Bilds angeordnet. Das durch die Blende hindurchtretende Licht wird mittels eines dispersiven Elements aufgefächert und mittels eines Kamerasensors aufgenommen. Dadurch kann von dem Kamerasensor eine Vielzahl von Spektren mit jeweils zugeordneter räumlicher Koordinate entlang der Längsrichtung der schlitzförmigen Blende aufgenommen werden. Die beschriebene Vorrichtung ist weiterhin dazu eingerichtet, in einer von der Längsrichtung der schlitzförmigen Blende verschiedenen Richtung weitere Spektren entlang der Längsrichtung der schlitzförmigen Blende aufzunehmen. Das dieser Offenbarung zugrunde liegende Verfahren zur Erzeugung von Multispektral- oder Hyperspektralbildern ist auch als sogenanntes Pushbroom-Verfahren bekannt.

Neben dem Pushbroom-Verfahren gibt es weitere Verfahren zur Erzeugung von Multispektral- oder Hyperspektralbildern. Beim sogenannten Whiskbroom-Verfahren wird das Untersuchungsgebiet oder auch Objekt punktweise abgefahren und für jeden Punkt ein Spektrum gewonnen. Im Gegensatz dazu, werden bei dem Staring-Verfahren mehrere Bilder mit denselben räumlichen Koordinaten aufgenommen. Dabei werden von Bild zu Bild verschiedene Spektralfilter und/oder Beleuchtungsquellen verwendet, um spektrale Information aufzulösen. Ferner gibt es Verfahren, gemäß denen durch geeignete optische Elemente wie optische Slicer, Linsen und Prismen ein zweidimensionales Mehrfarbenbild in mehrere spektrale Einzelbilder zerlegt wird, die gleichzeitig auf unterschiedlichen Detektoren oder Detektorbereichen erfasst werden. Dies wird bisweilen als Schnappschuss-Verfahren bezeichnet.

Wie in DE 10 2020 105 458 A1 beschrieben, eignen sich multispektrale und hyperspektrale Bildgebungsvorrichtungen insbesondere als endoskopische Bildgebungsvorrichtung. In dem Zusammenhang ist multispektrale und/oder hyperspektrale Bildgebung ein fundamentales Einsatzfeld beispielsweise zur Diagnostik sowie zur Beurteilung eines Erfolgs bzw. einer Qualität eines Eingriffs.

Daneben wird insbesondere im medizinischen Bildgebungsbereich Weißlichtbildgebung eingesetzt. Beobachtetes Gewebe wird mit Weißlicht beleuchtet, und mittels einer Kamera oder anderer Bilderfassungssensorik werden Bilder des Gewebes erzeugt, die dann einem Benutzer angezeigt werden können.

Ferner wird Fluoreszenzbildgebung eingesetzt, im Speziellen im medizinischen Bildgebungsbereich. Gewebe wird dabei in einem bestimmten Wellenlängenbereich beleuchtet, um gezielt in bestimmte Entitäten wie beispielsweise Gewebebereiche eingebrachte fluoreszierende Farbstoffmoleküle anzuregen. Das daraufhin emittierte Licht mit größerer Wellenlänge kann durch einen geeignet gewählten Filter beobachtet werden, mittels dessen Anregungslicht ausgeblendet werden kann.

Typische Fluoreszenzfarbstoffe weisen häufig ein ähnliches Anregungsspektrum auf, sodass sie grundsätzlich mit der gleichen Bildgebungsvorrichtung verwendbar sind. Jedoch benötigen verschiedene Fluoreszenzfarbstoffe in der Regel unterschiedliche Sensitivitäten eines Kamerasystems und/oder unterschiedliche Beobachtungsfilter. Auch sind unterschiedliche Fluoreszenzfarbstoffe in ähnlichen Spektralbereichen nicht zeitgleich einsetzbar, um unterschiedliche Ziele zu visualisieren, beispielsweise zeitgleiche Visualisierung von Tumoren und Perfusion. Herkömmliche Kamerasysteme können solche Fluoreszenzfarbstoffe mit überlappenden Fluoreszenzspektren nicht zuverlässig unterscheiden. Durch Verwendung von Beobachtungsfilter im sichtbaren Wellenlängenbereich wird eine Weißlicht-Farbdarstellung erschwert beziehungsweise eine korrekte Farbwiedergabe verhindert.

Multimodale Bildgebungsvorrichtungen gestatten es, wahlweise Weißlichtbilder und/oder Multispektralbilder und/oder Fluoreszenzbilder und/oder Hyperspektralbilder aufzunehmen. Beispiele für derartige Bildgebungsvorrichtungen sind multimodale Endoskope und multimodale Exoskope. Zur Realisierung unterschiedlicher Modi können Beleuchtungsvorrichtungen erforderlich sein, die in unterschiedlichen Beleuchtungsmodi betreibbar sind, um bedarfsweise Beleuchtungslicht in unterschiedlichen Spektralbereichen zu erzeugen.

Aus US 10,481,095 B2 und US 11,668,922 B2 sind Bildgebungsvorrichtungen mit mehreren Beleuchtungsquellen bekannt, deren jeweils emittiertes Licht mittels Strahlteilerelementen zusammengeführt werden kann.

Ausgehend vom Stand der Technik liegt der Erfindung insbesondere aber nicht beschränkt darauf die Aufgabe zugrunde, eine Bildgebungsvorrichtung vorteilhaft weiterzuentwickeln.

Diese Aufgabe wird erfindungsgemäß gelöst durch eine Bildgebungsvorrichtung und ein Verfahren zum Betrieb einer Bildgebungsvorrichtung, wie sie hierin beschrieben und in den Ansprüchen definiert sind.

Die Erfindung betrifft eine Bildgebungsvorrichtung, insbesondere eine endoskopische, exoskopische und/oder mikroskopische Bildgebungsvorrichtung, umfassend:
- eine Beleuchtungsvorrichtung, welche zur Beleuchtung eines Untersuchungsbereichs vorgesehen ist,
- eine Bildaufnahmeeinheit, welche eine Hyperspektralsensorik zur Aufnahme zumindest eines hyperspektralen Abbilds des Untersuchungsbereichs aufweist, und
- eine Analyseeinheit zur Analyse des Abbilds.

In einem Aspekt der Erfindung, welcher für sich allein genommen aber auch in Kombination mit dem anderen Aspekt der Erfindung betrachtet werden kann, kann die Beleuchtungsvorrichtung zur gleichzeitigen Anregung eines ersten Lumineszenzfarbstoffs und zumindest eines zweiten Lumineszenzfarbstoffs vorgesehen sein.

In einem weiteren Aspekt der Erfindung, welcher für sich allein genommen aber auch in Kombination mit dem anderen Aspekt der Erfindung betrachtet werden kann, kann die Analyseeinheit dazu vorgesehen ist, ein erstes Lumineszenzsignal eines ersten Lumineszenzfarbstoffs und zumindest ein zweites Lumineszenzsignal zumindest eines zweiten Lumineszenzfarbstoffs in dem Abbild zu unterscheiden, vorzugsweise zu identifizieren.

Die Erfindung betrifft ferner ein Verfahren zum Betrieb einer Bildgebungsvorrichtung, insbesondere einer endoskopischen, exoskopischen und/oder mikroskopischen Bildgebungsvorrichtung, wobei ein Untersuchungsbereichs bestrahlt wird und dabei gleichzeitig ein erster Lumineszenzfarbstoff und zumindest ein zweiter Lumineszenzfarbstoff angeregt werden und wobei ein hyperspektrales Abbild des Untersuchungsbereichs aufgenommen wird.

Bei den Lumineszenzfarbstoffen kann es sich jeweils um einen Phosphoreszenzfarbstoff oder vorteilhaft um einen Fluoreszenzfarbstoff handeln, welcher einem Gewebe zugesetzt sein kann, beispielsweise Cyanin 5.5 (Cy 5.5), Indocyaningrün (ICG), S0456, ZW800-1, IRDye800, BM104, 5-FAM oder Fluoreszin und/oder Fluoreszenzfarbstoffe mit Anregung im roten oder blauen Wellenlängenbereich, oder welcher natürlich in einem Gewebe vorkommt.

Durch die obengenannten Merkmale kann eine Bildgebungsvorrichtung vorteilhaft weiterentwickelt werden, insbesondere können mehrere Lumineszenzen, insbesondere Floreszenzen, vorteilhaft gleichzeitig zum Einsatz kommen und/oder es kann auf einen Wechsel einer Optik, insbesondere während eines Eingriffs und/oder einer Operation, verzichtet werden. Insbesondere können unterschiedliche Lumineszenzfarbstoffe, insbesondere Fluoreszenzfarbstoffe, verschiedener klinischer Marker unterschieden und insbesondere identifiziert werden, insbesondere um dann eine automatische Anwahl eines entsprechenden Lumineszenzbetriebsmodus, insbesondere Fluoreszenzbetriebsmodus, vorteilhaft mit entsprechend für den Lumineszenzfarbstoff, insbesondere Fluoreszenzfarbstoff, erforderlicher Sensitivität einer Sensorik und/oder entsprechenden Darstellungseigenschaften einer Anzeigeeinheit auszuwählen. Hierdurch kann eine zeitgleiche und/oder reaktionsschnelle medizinische Visualisierung ermöglicht werden, beispielsweise von Tumoren und Perfusion. Dies kann sowohl aus regulatorischer Sicht als auch seitens eines Verkaufsmodells vorteilhaft sein. Vor dem Hintergrund, dass abzusehen ist, dass eine Vielzahl von Fluoreszenzmarkersubstanzen eine klinische Zulassung erhalten werden, kann eine bestehende Bildgebungsvorrichtung für diese neuen Fluoreszenzmarkersubstanzen adaptiert werden, beispielsweise durch ein entsprechendes Softwareupdate und/oder durch eine bestehende Netzwerkverbindung zu einem Server, welcher eine Datenbank der zugelassenen Fluoreszenzmarkersubstanzen aufweist, und/oder durch Austausch eines optischen Filters. Durch Verwendung einer Hyperspektralsensorik kann eine Anregungslicht vorteilhaft von einem Lumineszenzlicht, insbesondere einem Fluoreszenzlicht, getrennt werden, sodass insbesondere kein Beobachtungsfilter erforderlich ist.

Unter "vorgesehen" soll speziell ausgestattet, eingerichtet und/oder programmiert verstanden werden und insbesondere nicht eine bloße Eignung. Für Objekte in einem Abbild des Untersuchungsbereichs werden der Einfachheit halber dieselben Begriffe verwendet wie für die entsprechenden Objekte im Untersuchungsbereich.

Die Bildgebungsvorrichtung kann bevorzugt eine medizinische Bildgebungsvorrichtung sein. Die Bildgebungsvorrichtung kann neben der Beleuchtungsvorrichtung ein Bildgebungsgerät, beispielsweise ein Endoskop, Exoskop und/oder Mikroskop umfassen, das mit einer optischen Schnittstelle der Beleuchtungsvorrichtung optisch verbindbar sein kann. Die optische Schnittstelle kann wahlweise lösbar und verbindbar sein. Zudem kann die optische Schnittstelle mit einer mechanischen Schnittstelle kombiniert sein, sodass eine optische Verbindung beispielsweise automatisch dann hergestellt wird, wenn das Bildgebungsgerät mechanisch angekoppelt wird.

Die Bildgebungsvorrichtung und insbesondere das Bildgebungsgerät ist in einigen Ausführungsformen dazu eingerichtet, zur Begutachtung und/oder Beobachtung in einen Hohlraum einführbar zu sein, beispielsweise in eine künstliche und/oder natürliche Kavität, etwa in ein Inneres eines Körpers, in ein Körperorgan, in Gewebe oder dergleichen. Die Bildgebungsvorrichtung und insbesondere das Bildgebungsgerät kann auch dazu eingerichtet sein, zur Begutachtung und/der Beobachtung in ein Gehäuse, eine Verschalung, einen Schacht, ein Rohr oder eine andere, insbesondere künstliche, Struktur einführbar zu sein. Die Bildgebungsvorrichtung und insbesondere das Bildgebungsgerät kann dazu eingerichtet sein, Gewebeparameter, Bilder von Wunden, Bilder von Körperteilen etc. aufzunehmen. Beispielsweise kann die Bildgebungsvorrichtung dazu eingerichtet sein, ein Operationsfeld abzubilden.

Die Beleuchtungsvorrichtung ist zur Bereitstellung von Beleuchtungslicht für das Bildgebungsgerät vorgesehen. Die Beleuchtungsvorrichtung kann multimodal ausgebildet und/oder in mehreren unterschiedlichen Beleuchtungsmodi betreibbar sein. Die Beleuchtungsvorrichtung kann dazu ausgebildet sein, ein breites, insbesondere kontinuierliches, Emissionsspektrum oder Licht aus einem schmalbandigen Wellenlängenbereich oder aus mehreren schmalbandigen Wellenlängenbereichen bereitzustellen.

Die Beleuchtungsvorrichtung kann mehrere unabhängig voneinander wahlweise aktivierbare Leuchtquellen umfassen, die dazu eingerichtet sind, Licht unterschiedlicher Emissionsspektren zu emittieren, um das Beleuchtungslicht zu liefern. Die Beleuchtungsvorrichtung kann in zumindest einem Weißlichtmodus betreibbar sein, in dem eine erste Gruppe der Leuchtquellen zumindest zeitweise aktiviert ist und in dem die Beleuchtungsvorrichtung Beleuchtungslicht für Weißlichtbildgebung liefert. Ferner kann die Beleuchtungsvorrichtung in zumindest einem Lumineszenzmodus, insbesondere einem Fluoreszenzmodus, betreibbar sein, in dem eine zweite Gruppe der Leuchtquellen zumindest zeitweise aktiviert ist und in dem die Beleuchtungsvorrichtung Beleuchtungslicht für Lumineszenzbildgebung, insbesondere Fluoreszenzbildgebung, liefert, welches insbesondere in einem Anregungswellenlängenbereich anzuregender Lumineszenzfarbstoffe, insbesondere Fluoreszenzfarbstoffe, eine hohe Intensität aufweist. Zudem kann die Beleuchtungsvorrichtung in einem Hybridmodus betreibbar sein, in welchem die Beleuchtungsvorrichtung dazu vorgesehen sein kann, Weißlicht zur Beleuchtung des Untersuchungsbereichs gleichzeitig und/oder sequenziell zu einem Anregungslicht zur Anregung der zumindest zwei Lumineszenzfarbstoffe bereitzustellen, wodurch Weißlichtbildgebung und Lumineszenzbildgebung vorteilhaft kombiniert werden können. Die Leuchtquellen können zumindest eine Leuchtquelle umfassen, welche sowohl in der ersten Gruppe als auch in der zweiten Gruppe enthalten ist. Insbesondere können die Gruppen auch nur eine einzelne Lichtquelle aufweisen.

Die Leuchtquellen können insbesondere einfarbige LEDs (Leuchtdioden) und/oder Laserdioden umfassen. Ferner kann zumindest eines der Leuchtquellen eine Weißlicht-LED, insbesondere aufweisend eine Phosphorbeschichtung, oder eine andere Weißlichtquelle sein, beispielsweise bestehend aus einem Zusammenschluss mehrerer einfarbiger LEDs, welche gemeinsam das Weißlicht erzeugen. In einigen Ausführungsformen umfasst die Beleuchtungsvorrichtung zumindest eine blaue Leuchtquelle, zumindest eine rote Leuchtquelle, zumindest eine dunkelrote Leuchtquelle und zumindest eine Nah-IR-Leuchtquelle (Nahinfrarot-Leuchtquelle), beispielsweise mit einer Emission im Wellenlängenbereich von 750 nm bis 800 nm.

Die Beleuchtungsvorrichtung kann mehrere dichroitisch kombinierte LEDs umfassen, insbesondere zur Anregung von visueller blauer Fluoreszenz (ca. 460 nm Anregung), visueller roter Fluoreszenz (ca. 660 nm Anregung) und nahinfraroter Fluoreszenz (ca. 780 nm Anregung). Die Beleuchtungsvorrichtung erlaubt in manchen Ausgestaltungen eine beliebige Mischung der Lichtintensitäten der Leuchtquellen. Zudem wäre denkbar, dass die Beleuchtungsvorrichtung zumindest einen Anregungsfilter aufweist, um ein Anregungslicht für einen der Lumineszenzfarbstoffe so einzuschränken, dass dieses nicht mit dem Lumineszenzspektrum eines anderen der Lumineszenzfarbstoffe oder aller anderen Lumineszenzfarbstoffe überlappt.

In einigen Anwendungen kann vorgesehen sein, dass die Beleuchtungsvorrichtung sequenziell nacheinander unterschiedliche Beleuchtungen bereitstellt, sodass beispielsweise während eines ersten Zeitintervalls ein Anregungslicht für die Lumineszenzfarbstoffe und während eines zweiten Zeitintervalls Weißlicht bereitgestellt wird. Die Bildaufnahmeeinheit kann dann während der jeweiligen Zeitintervalle räumlich aufgelöste Abbilder des Untersuchungsbereichs bereitstellen.

In manchen Ausgestaltungen der Erfindung kann die Beleuchtungsvorrichtung eine einzige Beleuchtungsquelle aufweisen, welche zur gleichzeitigen Anregung der zumindest zwei Lumineszenzfarbstoffe vorgesehen ist. Hierdurch kann eine Komplexität und/oder eine Bauteilevielfalt und/oder ein Platzbedarf vorteilhaft reduziert werden. Zudem können Kosten reduziert werden.

Darunter, dass die Beleuchtungsvorrichtung und/oder die Beleuchtungsquelle "zur gleichzeitigen Anregung eines ersten Lumineszenzfarbstoffs und zumindest eines zweiten Lumineszenzfarbstoffs" vorgesehen sein soll, soll verstanden werden, dass die Beleuchtungsvorrichtung und/oder die Beleuchtungsquelle in zumindest einem Betriebsmodus, insbesondere im Lumineszenzmodus und/oder im Hybridmodus, ein Anregungslicht emittiert, welches zu einer zeitgleichen und/oder sequenziellen Anregung der zumindest zwei Lumineszenzfarbstoffe geeignet ist.

Die Bildaufnahmeeinheit und/oder die Analyseeinheit kann zumindest teilweise Teil des Bildgebungsgeräts sein, derart, dass einzelne Elemente der Bildaufnahmeeinheit und/oder der Analyseeinheit Bestandteil des Bildgebungsgeräts sind. Alternativ kann die Bildaufnahmeeinheit und/oder Analyseeinheit jedoch auch als eine von dem Bildgebungsgerät separate, beispielsweise mittels eines Lichtleiters und/oder einer Kabelverbindung mit dem Bildgebungsgerät verbindbare oder verbundene, Einheit bereitgestellt sein.

Die Bildaufnahmeeinheit stellt räumliche und spektrale Informationen bereit. Die Bildaufnahmeeinheit ist räumlich und spektral auflösend ausgebildet und kann zumindest eine Optik umfassen, die mit der Hyperspektralsensorik gekoppelt ist. Die Hyperspektralsensorik ist dazu eingerichtet, eine Bilderfassung eines Bildbereichs durchzuführen, bei der räumliche und spektrale Bilddaten erzeugt werden. Die so erzeugten hyperspektralen Abbilder weisen sowohl räumliche als auch spektrale Informationen auf. Hyperspektrale Bildgebung bzw. hyperspektrale Abbilder kann sich dabei insbesondere auf solche Bildgebung beziehen, bei der wenigstens 20, wenigstens 50 oder sogar wenigstens 100 Spektralbänder voneinander unabhängig erfassbar sind und/oder erfasst werden.

Die Hyperspektralsensorik kann dabei nach dem Whiskbroom-Verfahren und/oder nach dem Schnappschuss-Verfahren und/oder bevorzugt nach dem Pushbroom-Verfahren arbeiten.

Es wäre denkbar, dass die Hyperspektralsensorik einen Zeilensensor aufweist, welcher eine Zeile eines Abbilds mit einer einzelnen ersten Raumkoordinate x erfasst, wobei zu jedem Bildpunkt der Zeile zusätzlich Spektraldaten aufgenommen werden, sodass der Zeilensensor insbesondere ein (x; λ)-Feld aufnimmt. Zur Aufnahme der Spektraldaten wird das Licht durch eine Schlitzblende des Zeilensensors geführt und dann spektral aufgespalten, beispielsweise durch ein Prisma und/oder ein optisches Gitter, insbesondere ein Gitterprisma und vorzugsweise eine "prism-grating-prism" (PGP)-Anordnung, des Zeilensensors. Beispielsweise kann der Zeilensensor dazu ausgebildet sein, den Wellenlängenbereich von 500 nm bis 1000 nm mit einer Schrittweite von 5 nm aufzunehmen. Die zur ersten Raumkoordinate senkrechte zweite Raumkoordinate y des Abbilds kann durch eine Bewegung des Zeilensensors und/oder der Schlitzblende erreicht werden, beispielsweise manuell durch einen Bediener, insbesondere einen Arzt, und/oder bevorzugt automatisch durch eine Scaneinheit der Hyperspektralsensorik, insbesondere einen Pushbroom-Scanner. Durch Verwendung einer Hyperspektralsensorik nach dem Pushbroom-Verfahren kann eine vorteilhaft hohe spektrale Auflösung mit einer relativ schnellen Bildaufnahme kombiniert werden.

Die Bildaufnahmeeinheit kann in einigen Ausgestaltungen eine Filtereinheit aufweisen, mittels welcher bestimmte Lichtanteile aus dem Untersuchungsbereich herausgefiltert werden können, beispielsweise ein Anregungslicht und/oder bestimmte Lichtanteile eines Lumineszenzlichts, insbesondere Fluoreszenzlichts. Die Filtereinheit kann dabei ansteuerbar sein, insbesondere zur Änderung von Filtereigenschaften.

In einigen Ausgestaltungen kann die Bildaufnahmeeinheit und/oder das Bildgebungsgerät eine Breitbandoptik aufweisen, welche an einem Eingang der Hyperspektralsensorik angeordnet ist. Die Bildaufnahmeeinheit und/oder das Bildgebungsgerät und insbesondere die Breitbandoptik ist vorzugsweise frei von Beobachtungsfilter, und zwar insbesondere frei von Beobachtungsfiltern zum Blockieren eines Anregungslichts. Das Bildgebungsgerät und/oder die Breitbandoptik weist bevorzugt eine breitbandige Transmission im ultravioletten, sichtbaren und/oder nah- und "short wave"-infraroten Wellenlängenbereich auf. Das Bildgebungsgerät und/oder die Breitbandoptik umfassen beispielsweise eine hohe Transmission im Wellenlängenbereich von 400 nm bis 1000 nm oder alternativ in den Wellenlängenbereichen von 400 nm bis 700 nm und von 800 nm bis 1000 nm, wobei eine Transmission im Bereich von 700 nm bis 800 nm gering ist. Hierdurch kann eine Anwendungsflexibilität gesteigert werden. So kann vorteilhaft ein Hybridmodus bereitgestellt werden, bei welchem Weißlichtbildgebung und Lumineszenzbildgebung gleichzeitig auftreten. Ferner kann eine Komplexität und/oder eine Bauteilevielfalt und/oder ein Bauraumbedarf reduziert werden. Zudem können Kosten reduziert werden. Des Weiteren kann das Anregungslicht für zusätzlichen Analysen herangezogen werden.

Die Bildgebungsvorrichtung kann eine Steuerung umfassen, die dazu eingerichtet ist, einen Betriebszustand des Bildgebungsgeräts und/oder der Bildaufnahmeeinheit und einen Beleuchtungsmodus der Beleuchtungsvorrichtung automatisch aufeinander abzustimmen. Die Steuerung kann dazu eingerichtet sein, die Beleuchtungsvorrichtung und/oder das Bildgebungsgerät und/oder die Bildaufnahmeeinheit anzusteuern. Die Analyseeinheit kann Teil dieser Steuerung und/oder in dieser integriert sein.

Darunter, dass die Analyseeinheit dazu vorgesehen ist, die zumindest zwei Lumineszenzfarbstoffe in dem Abbild "zu unterscheiden", soll verstanden werden, dass die Analyseeinheit dazu eingerichtet ist, anhand zumindest einer Eigenschaft zugehöriger Lumineszenzspektren die zumindest zwei Lumineszenzfarbstoffe in dem Abbild zu unterscheiden, und zwar insbesondere dahingehend, dass es sich um zumindest zwei Lumineszenzfarbstoffe verschiedenen Typs handelt und/oder wo die zumindest zwei Lumineszenzfarbstoffe in dem zumindest einen Abbild des Bildsatzes angeordnet sind. Zur Unterscheidung der Lumineszenzfarbstoffe kann die Analyseeinheit eine Position eines Maximums eines Lumineszenzsignals und/oder eine mittlere Wellenlänge eines Lumineszenzsignals in einem Lumineszenzbereich ermitteln und/oder ein "unmixing"-Verfahren oder ähnliche, dem Fachmann als sinnvoll erscheinende Verfahren nutzen. Die aufgenommenen Lumineszenzsignale können dabei mittels eines "Standard-Normal-Variante" (SNV)-Verfahrens, eines Weißabgleichs oder eines anderen, dem Fachmann als sinnvoll erscheinenden Verfahrens durch die Analyseeinheit normiert werden. Zudem kann vorgesehen sein, dass die Analyseeinheit die Lumineszenzsignale über einen jeweiligen Wellenlängenbereich einer Lumineszenz mittelt. Die Unterscheidung durch die Analyseeinheit kann dabei einmalig, fortlaufend oder auch in einem speziellen Testschritt, insbesondere auch wiederkehrend, erfolgen.

Darunter, dass die Analyseeinheit dazu vorgesehen ist, die zumindest zwei Lumineszenzfarbstoffe in dem Abbild "zu identifizieren", soll verstanden werden, dass die Analyseeinheit dazu eingerichtet ist, anhand zumindest einer Eigenschaft zugehöriger Lumineszenzspektren die Art der zumindest zwei Lumineszenzfarbstoffe zu erkennen.

Bevorzugt ist die Analyseeinheit dazu vorgesehen, die zumindest zwei Lumineszenzsignale mit Referenzlumineszenzspektren verschiedener Lumineszenzfarbstoffe zu vergleichen. Die Analyseeinheit kann beispielsweise dazu vorgesehen sein, bestimmte Eigenschaften der aufgezeichneten Lumineszenzspektren jeweils mit entsprechenden Referenzeigenschaften eines Referenzlumineszenzspektrums zu vergleichen und/oder die aufgezeichneten Lumineszenzspektren jeweils mit Referenzlumineszenzspektren zu vergleichen. Zur Identifizierung der Lumineszenzfarbstoffe kann die Analyseeinheit eine Position eines Maximums eines Lumineszenzsignals und/oder eine mittlere Wellenlänge eines Lumineszenzsignals in einem Lumineszenzbereich ermitteln und/oder ein "unmixing"-Verfahren oder ähnliche, dem Fachmann als sinnvoll erscheinende Verfahren nutzen. Die aufgenommenen Lumineszenzsignale können dabei mittels eines "Standard-Normal-Variante" (SNV)-Verfahrens, eines Weißabgleichs oder eines anderen, dem Fachmann als sinnvoll erscheinenden Verfahrens durch die Analyseeinheit normiert werden. Zudem kann vorgesehen sein, dass die Analyseeinheit die Lumineszenzsignale über einen jeweiligen Wellenlängenbereich einer Lumineszenz mittelt.

Insbesondere kann die Analyseeinheit für jeden Pixel der Hyperspektralsensorik ein dort anliegendes Lumineszenzsignal analysieren und dieses mittels gängiger Methoden in seine einzelnen Bestandteile zerlegen und diese mit den Referenzlumineszenzspektren vergleichen. Alternativ oder zusätzlich wäre denkbar, dass die Analyseeinheit für jeden Pixel der Hyperspektralsensorik ein dort anliegendes Lumineszenzsignal auf seine Maxima hin untersucht und diese Maxima mit den Maxima der Referenzlumineszenzspektren vergleicht. Hierdurch kann eine besonders zuverlässige Unterscheidung, insbesondere Identifizierung, ermöglicht werden.

Die Analyseeinheit kann eine Speichereinheit aufweisen, in welcher die entsprechenden Eigenschaften des Referenzlumineszenzspektrums und/oder das Referenzlumineszenzspektrum als solches abgespeichert sind, beispielsweise tabellarisch. Alternativ oder zusätzlich kann die Analyseeinheit eine Kommunikationsschnittstelle für eine Kommunikation über ein Netzwerk, insbesondere dem Internet, mit einer Datenbank aufweisen, in welcher die entsprechenden Eigenschaften des Referenzlumineszenzspektrums und/oder das Referenzlumineszenzspektrum hinterlegt sind, beispielsweise tabellarisch, wodurch automatisch eine Aktualisierung der Auswertemöglichkeiten hinsichtlich neu zugelassener Fluoreszenzmarker erfolgen kann. Die Identifizierung durch die Analyseeinheit kann dabei einmalig, fortlaufend oder auch in einem speziellen Testschritt, insbesondere auch wiederkehrend, erfolgen.

Bevorzugt ist die Analyseeinheit dazu vorgesehen, die zumindest zwei Lumineszenzsignale auch bei sich teilweise überlappenden Lumineszenzspektren der zumindest zwei Lumineszenzfarbstoffe zu unterscheiden, vorzugsweise zu identifizieren, wodurch ein Einsatzbereich der Bildgebungsvorrichtung besonders vorteilhaft gesteigert werden kann. Dies ist insbesondere durch Verwendung einer hyperspektralen Bildgebung möglich.

Vorteilhaft ist die Analyseeinheit dazu vorgesehen, die zumindest zwei Lumineszenzsignale auch bei einem gleichzeitig im Abbild vorhandenen Weißlichtbild des Untersuchungsbereichs zu unterscheiden, vorzugsweise zu identifizieren, wodurch vorteilhaft ein hybrider Betriebsmodus, insbesondere der zuvor genannte Hybridmodus, bereitgestellt werden kann.

Bevorzugt kann die Analyseeinheit dazu vorgesehen sein, zumindest eine Positionsinformation der zumindest zwei Lumineszenzfarbstoffe zu berücksichtigen.

Diesbezüglich kann die Analyseeinheit in machen Ausgestaltungen dazu vorgesehen sein, bei einer Auswertung eines Lumineszenzsignals in einem Bereich des Abbilds zumindest ein Lumineszenzsignal in einem Umgebungsbereich des Bereichs zu berücksichtigen, wodurch eine Unterscheidung, insbesondere Identifizierung, der Lum ineszenzfarbstoffe verbessert werden kann, insbesondere im Falle eines ungünstigen Signal-Rauschverhältnisses in dem Bereich. Wenn beispielsweise in einem unmittelbar den Bereich angrenzenden Umgebungsbereich ein bestimmter Lumineszenzfarbstoff erkannt wurde, ist mit großer Wahrscheinlichkeit davon auszugehen, dass in dem Bereich ebenfalls dieser Lumineszenzfarbstoff anzutreffen ist. Ferner kann in einigen Ausgestaltungen vorgesehen sein, ein spektrales Signal im Abbild räumlich zu mitteln, da die Fluoreszenzverteilung häufig sehr großflächig im Abbild ist.

Ferner kann die Analyseeinheit dazu vorgesehen sein, bei einer Auswertung eines Lumineszenzsignals in einem Bereich des Abbilds typische biologische Formen zu berücksichtigen, wodurch eine Unterscheidung, insbesondere Identifizierung, der Lum ineszenzfarbstoffe weiter verbessert werden kann. So sind Nervenbahnen oder Blutgefäße beispielsweise eher länglich, was bei der Unterscheidung und insbesondere Identifizierung, von Lumineszenzfarbstoffen hilfreich sein kann, insbesondere wenn zusätzlich berücksichtigt wird, wo sich diese in einem Gewebe üblicherweise anreichern.

In einigen Ausgestaltungen umfasst die Bildgebungsvorrichtung eine Anzeigeeinheit zur Darstellung einer Ansicht wenigstens eines Teils des Untersuchungsbereichs, wobei die Analyseeinheit dazu vorgesehen ist, die zumindest zwei Lumineszenzsignale über die Anzeigeeinheit in der Ansicht gemeinsam als verschiedenartig, vorzugsweise verschiedenfarbig, gekennzeichnete Overlays über dem Weißlichtbild darzustellen. Hierdurch kann eine Bedienbarkeit besonders vorteilhaft gesteigert werden. Insbesondere kann eine Informationsvermittlung optimiert werden. Die Overlays sind bevorzugt einzeln zu- und abschaltbar. Dabei könnte die Darstellung der Overlays und/oder des Weißlichtbilds unabhängig voneinander durch einen Bediener änderbar, vorzugsweise frei wählbar sein. Insbesondere könnte eine Farbe, eine Helligkeit, ein Kontrast, eine Durchsichtigkeit, ein Muster, ein Umriss und/oder ein weiterer, dem Fachmann als sinnvoll erscheinender Darstellungsparameter durch einen Bediener änderbar und/oder wählbar sein. Ferner wäre denkbar, dass jeder Bediener für sich ein entsprechendes Darstellungsprofil erstellen und für spätere Anwendungen abspeichern kann, beispielsweise lokal oder über eine Netzwerkverbindung auf einem Server, wodurch die Einstellungen auch an verschiedenen Bildgebungsvorrichtungen verfügbar gemacht werden können.

Die Analyseeinheit und/oder die Bildaufnahmeeinheit kann dazu vorgesehen sein, zumindest einen Belichtungsparameter der Hyperspektralsensorik so einzustellen, dass zumindest ein Lumineszenzsignal eines Lumineszenzfarbstoffs, vorzugsweise sämtliche Lumineszenzsignale aller Pixel der Hyperspektralsensorik, wesentlich ausgesteuert ist/sind, wobei das Lumineszenzsignal beziehungsweise die Lum ineszenzsignale dabei vorzugsweise zwischen 50 % und 100 %, insbesondere zwischen 75 % und 100 %, eines gesamten Detektionsbereichs der Hyperspektralsensorik abdeckt/abdecken. Hierdurch kann eine Aufnahme des Abbilds vorteilhaft verbessert werden. Insbesondere kann ein vorteilhaftes Signal-Rauschverhältnis erreicht werden. Zumindest ein aus dem Untersuchungsbereich stammendes Reflektionssignal des Anregungslicht kann dabei die Hyperspektralsensorik übersteuern und/oder größer als der Detektionsbereich sein. Bevorzugt decken sämtliche auftretende Lumineszenzsignale den Detektionsbereich teilweise ab und übersteuern die Hyperspektralsensorik insbesondere nicht. Die Analyseeinheit und/oder die Bildaufnahmeeinheit kann dazu vorgesehen sein, den zumindest einen Belichtungsparameter im laufenden Betrieb anzupassen, insbesondere für jeweils folgende Bildaufnahmen und/oder Frames.

In einigen Ausgestaltungen der Erfindung kann die Beleuchtungsvorrichtung dazu vorgesehen sein, zumindest eine Information hinsichtlich eines zur Beleuchtung des Untersuchungsbereichs verwendeten Beleuchtungsspektrums an die Bildaufnahmeeinheit und/oder an die Analyseeinheit zu übermitteln. Hierdurch kann eine Bildaufnahme vorteilhaft verbessert werden. Bevorzugt ist die Beleuchtungsvorrichtung dazu vorgesehen, das gesamte Beleuchtungsspektrum zu übermitteln, wodurch insbesondere im Falle eines Wechsels und/oder eines Neueinbaus einer Lichtquelle der Beleuchtungsvorrichtung oder der Beleuchtungsvorrichtung als Ganzes eine entsprechende dazu passende Einstellung der Bildaufnahmeeinheit und/oder der Analyseeinheit erfolgen kann, beispielsweise hinsichtlich des Detektionsbereichs.

Die erfindungsgemäßen Vorrichtungen und Systeme sowie das erfindungsgemäße Verfahren sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf eine Vorrichtung beschriebenen Merkmale und Eigenschaften, aber auch Verfahrensweisen, sinngemäß auf Verfahren übertragbar und im Sinne der Erfindung einsetzbar und als mitoffenbart gelten. Gleiches gilt auch in umgekehrter Richtung. Das bedeutet, dass auch in Bezug auf Verfahren genannte, bauliche also vorrichtungsgemäße Merkmale im Rahmen der Vorrichtungsansprüche berücksichtigt, beansprucht und ebenfalls zur Offenbarung gezählt werden können.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist gegebenenfalls nur eines davon in den Figuren und in der Beschreibung mit einem Bezugszeichen versehen. Die Beschreibung dieses Exemplars kann entsprechend auf die anderen Exemplare des Objekts übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine Bildgebungsvorrichtung mit einem Bildgebungsgerät in Form eines Endoskops zur Aufnahme eines Untersuchungsbereichs und mit einer Bildaufnahmeeinheit, welche eine Hyperspektralsensorik aufweist,
- Fig. 2: eine schematische Darstellung der Hyperspektralsensorik,
- Fig. 3: zwei beispielhafte, durch die Bildaufnahmeeinheit aufgezeichnete Lum ineszenzsignale,
- Fig. 4: eine vergrößerte Darstellung eines Abbilds des Untersuchungsbereichs,
- Fig. 5: eine vereinfachte Darstellung der Lumineszenzsignale neben einem Reflektionssignal eines Anregungssignals und
- Fig. 6: ein Ablaufdiagramm eines Verfahrens zum Betrieb der Bildgebungsvorrichtung.

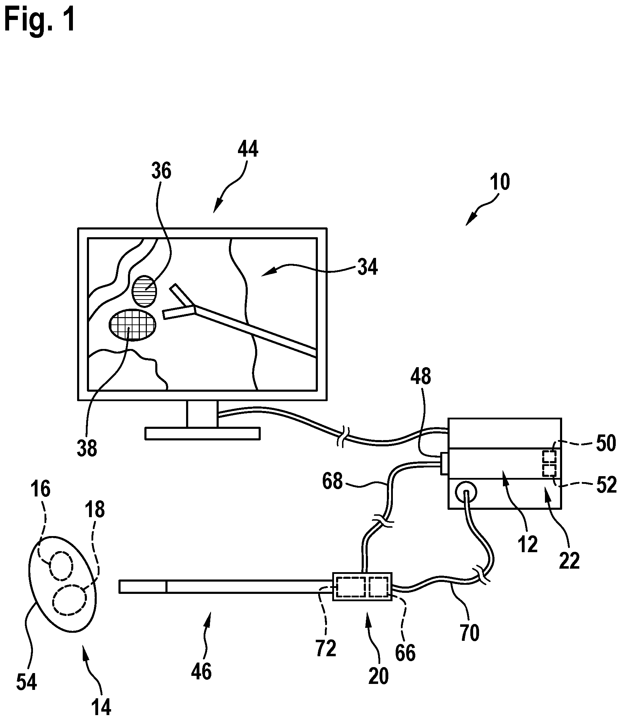

Fig. 1 zeigt eine schematische Darstellung einer Bildgebungsvorrichtung 10. Im exemplarisch dargestellten Fall ist die Bildgebungsvorrichtung 10 eine endoskopische Bildgebungsvorrichtung, konkret eine Endoskopvorrichtung. Alternativ könnte es sich bei der Bildgebungsvorrichtung 10 um eine exoskopische, eine mikroskopische oder eine makroskopische Bildgebungsvorrichtung handeln. Die Bildgebungsvorrichtung 10 ist beispielhaft als medizinische Bildgebungsvorrichtung gezeigt. Die Bildgebungsvorrichtung 10 ist beispielsweise zu einer Untersuchung einer Kavität vorgesehen. Die Bildgebungsvorrichtung 10 weist ein medizinisches Bildgebungsgerät 46 auf. Im dargestellten Fall handelt es sich hierbei um ein Endoskop.

Ferner umfasst die Bildgebungsvorrichtung 10 eine Beleuchtungsvorrichtung 12 mit einer optischen Schnittstelle 48, einer ersten Beleuchtungsquelle 50 und einer zweiten Beleuchtungsquelle 52. Das Bildgebungsgerät 46 ist optisch an die optische Schnittstelle 48 anbindbar, beispielsweise über einen Lichtleiter 68. Die optische Schnittstelle 48 kann Teil einer optisch-mechanischen Schnittstelle sein, die wahlweise lösbar und verbindbar ist. Das Bildgebungsgerät 46 kann wahlweise von der Beleuchtungsvorrichtung 12 abkoppelbar sein. Die Beleuchtungsquellen 50, 52 sind dazu eingerichtet, Beleuchtungslicht an die optische Schnittstelle 48 zu liefern. Bei einer Bildgebung mittels des Bildgebungsgeräts 46 können entsprechend die Beleuchtungsquellen 50, 52 das erforderliche Beleuchtungslicht bereitstellen, das zum Bildgebungsgerät 46 geführt und von dort auf ein abzubildendes Objekt, wie beispielsweise einen Situs, ausgekoppelt wird.

Die Bildgebungsvorrichtung 10 umfasst im dargestellten Fall ferner eine Anzeigeeinheit 44, auf welcher Bilder angezeigt werden können, die auf Bilddaten beruhen, die mittels des Bildgebungsgeräts 46 erfasst wurden. Hierbei kann es sich um Videobilder, Standbilder, Überlagerungen unterschiedlicher Bilder, Teilbilder, Bildsequenzen etc. handeln.

Die Bildgebungsvorrichtung 10 ist multimodal. Exemplarisch ist die Bildgebungsvorrichtung 10 in zwei grundlegenden Modi, einem Lumineszenzmodus und einem Weißlichtmodus, sowie in einem Hybridmodus betreibbar, in welchem die beiden zuvor genannten Modi gleichzeitig vorkommen.

Die Beleuchtungsvorrichtung 12 ist multimodal. Die Beleuchtungsvorrichtung 12 ist in unterschiedlichen Beleuchtungsmodi betreibbar, in denen sie Licht für unterschiedliche Bildgebungsmodi liefert. Vorliegend ist die Beleuchtungsvorrichtung 12 in zwei grundlegenden Modi, einem Lumineszenzmodus und einem Weißlichtmodus, sowie einem Hybridmodus betreibbar, in welchem die beiden zuvor genannten Modi gleichzeitig vorkommen. Ebenso ist das Bildgebungsgerät 46 in unterschiedlichen Betriebsmodi betreibbar, konkret ebenfalls einem Lumineszenzmodus und einem Weißlichtmodus sowie einem Hybridmodus.

Im entsprechenden Betriebsmodus der Bildgebungsvorrichtung 10 werden die Modi der Beleuchtungsvorrichtung 12 und des Bildgebungsgeräts 46 aufeinander abgestimmt.

Die Beleuchtungsvorrichtung 12 ist zur Beleuchtung eines Untersuchungsbereichs 14 vorgesehen.

Die Beleuchtungsvorrichtung 12 ist im Lumineszenzmodus und im Hybridmodus zur Anregung eines ersten Lumineszenzfarbstoffs 16 und zumindest eines zweiten Lumineszenzfarbstoffs 18 vorgesehen. Bei den Lumineszenzfarbstoffen 16, 18 handelt es sich vorliegend um Fluoreszenzfarbstoffe, die als Marker einem Gewebe 54 zugesetzt sein können oder in dem Gewebe 54 natürlich vorkommen können. Die Beleuchtungsvorrichtung 12 könnte zudem zur gleichzeitigen Anregung von mehr als zwei Lumineszenzfarbstoffen 16, 18 vorgesehen sein. Ferner könnte es sich bei den Lumineszenzfarbstoffen 16, 18 auch um Phosphoreszenzfarbstoffe handeln.

Die Beleuchtungsvorrichtung 12 ist dabei dazu vorgesehen, den ersten Lumineszenzfarbstoffs 16 und den zweiten Lumineszenzfarbstoffs 18 gleichzeitig anzuregen. Die Beleuchtungsquelle 50 ist zur Emission eines Anregungslichts mit einem Beleuchtungsspektrums vorgesehen, durch welches beide Lumineszenzfarbstoffe 16, 18 zeitgleich anregbar sind. Die Beleuchtungsquelle 50 kann als eine LED oder als eine Laserdiode ausgestalten sein.

Die Beleuchtungsvorrichtung 12 ist im Weißlichtmodus und im Hybridmodus dazu vorgesehen, Weißlicht zur Beleuchtung des Untersuchungsbereichs 14 bereitzustellen, und zwar im Hybridmodus gleichzeitig zum Anregungslicht zur Anregung der zumindest zwei Lumineszenzfarbstoffe 16, 18. Hierzu ist die Beleuchtungsquelle 52 vorgesehen, welche beispielsweise eine Weißlicht-LED und/oder mehrere LEDs aufweisen könnte, die im Verbund weißes Licht bereitstellen.

Die Bildgebungsvorrichtung 10 umfasst eine Bildaufnahmeeinheit 20. Diese ist vorliegend vollständig in dem Bildgebungsgerät 46 integriert. Alternativ könnte sie auch nur teilweise in dem Bildgebungsgerät 46 und teilweise in einer weiteren Einheit integriert sein, wobei das Bildgebungsgerät 46 und die weitere Einheit mittels eines Lichtleiters und/oder einer Kabelverbindung verbunden sein könnten (nicht dargestellt).

Die Bildaufnahmeeinheit 20 weist eine Hyperspektralsensorik 66 zur Aufnahme zumindest eines hyperspektralen Abbilds des Untersuchungsbereichs 14 auf. Die Hyperspektralsensorik 66 ist vollständig in dem Bildgebungsgerät 46 integriert. Alternativ könnte die Hyperspektralsensorik 66 jedoch auch vollständig in der weiteren Einheit integriert sein (nicht dargestellt).

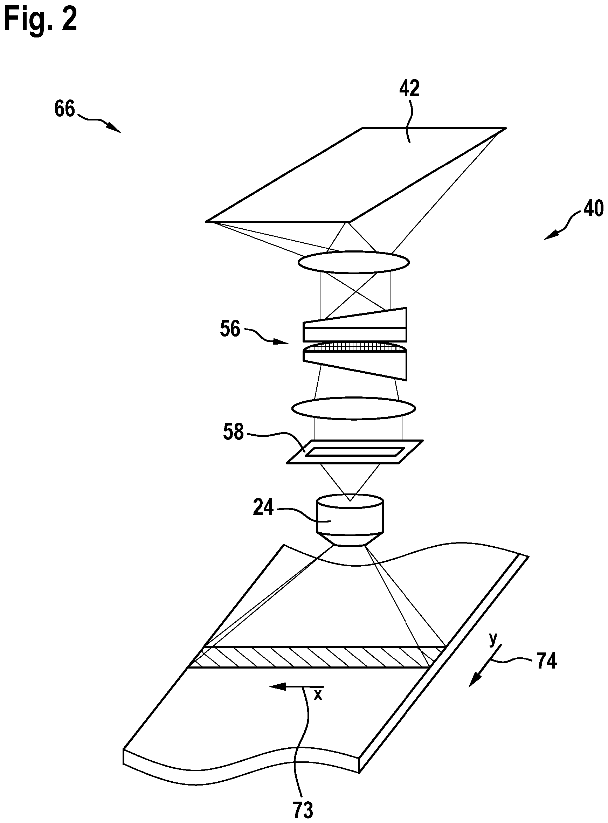

Die Hyperspektralsensorik 66 ist als eine Hyperspektralkamera ausgebildet. Die Hyperspektralsensorik 66 arbeitet nach dem Pushbroom-Prinzip und ist in Fig. 2 schematisch dargestellt.

Die Hyperspektralsensorik 66 umfasst einen Zeilensensor 40, welcher eine Zeile des Abbilds mit einer einzelnen ersten Raumkoordinate 73 erfasst, wobei zu jedem Bildpunkt der Zeile zusätzlich Spektraldaten aufgenommen werden, sodass der Zeilensensor 40 ein (x; λ)-Feld aufnimmt. Hierzu weist die Hyperspektralsensorik 66 eine Objektivlinse 24 und eine Schlitzblende 58 auf, um die Zeile auszuschneiden. Zur Aufnahme der Spektraldaten wird das Licht der Zeile durch einen PGP-Anordnung 56 des Zeilensensors 40 spektral aufgespalten und einem Matrixdetektor 42 des Zeilensensors 40 zugeführt. Die zur ersten Raumkoordinate 73 senkrechte zweite Raumkoordinate 74 des Abbilds wird durch eine Bewegung des Zeilensensors 40 aufgenommen. Hierzu weist die Hyperspektralsensorik 66 eine Scaneinheit auf, welche den gesamten Zeilensensor 40 entlang der zweiten Raumkoordinate 74 bewegt. Alternativ wäre auch denkbar, lediglich die Schlitzblende 58 zu bewegen und den Strahlengang mittels einer entsprechenden Optik anzupassen (nicht dargestellt).

Die Bildaufnahmeeinheit 20 weist eine Breitbandoptik 72 auf, welche in Fig. 1 lediglich schemenhaft wiedergegeben ist. Diese ist an einem Eingang der Hyperspektralsensorik 66 angeordnet. In der Breitbandoptik 72 umfasst in bekannter Weise optische Elemente, insbesondere Linsen, zur Bildgebung. Hierbei wird in der Breitbandoptik 72 bewusst auf die Verwendung eines Beobachtungsfilter zur Blockierung des Anregungslichts verzichtet, sodass der Hyperspektralsensorik 66 das gesamte Licht aus dem Untersuchungsbereich 14 zur Analyse zur Verfügung steht.

Die Beleuchtungsvorrichtung 12 ist dazu vorgesehen, zumindest eine Information hinsichtlich eines zur Beleuchtung des Untersuchungsbereichs 14 verwendeten Beleuchtungsspektrums an die Analyseeinheit 22 zu übermitteln. Diese Übermittlung kann einmalig, insbesondere zu Beginn eines Betriebsmodus oder nach dem Wechsel einer Lichtquelle 50, 52 der Beleuchtungsvorrichtung 12, und/oder beim Wechsel eines Betriebsmodus und/oder zeitlich, insbesondere periodisch, wiederkehrend erfolgen. Die Beleuchtungsvorrichtung ist dabei dazu vorgesehen, das gesamte Beleuchtungsspektrum zu übermitteln, wodurch im Falle eines Wechsels einer Lichtquelle eine entsprechende dazu passende Einstellung der Bildaufnahmeeinheit 20 und/oder der Analyseeinheit 22 erfolgen kann.

Die Bildgebungsvorrichtung 10 weist eine Analyseeinheit 22 zur Analyse der durch die Bildaufnahmeeinheit 20 erstellten Abbilder des Untersuchungsbereichs 14 auf. Vorliegend ist die Analyseeinheit über ein Kabel 70 mit der Bildaufnahmeeinheit 20 verbunden. Alternativ könnte die Analyseeinheit 22 auch teilweise oder vollständig in dem Bildgebungsgerät 46 integriert sein (nicht dargestellt). Die Analyseeinheit 22 kann ferner zu einer Kommunikation mit der Beleuchtungsvorrichtung 12 vorgesehen sein und diese entsprechend ansteuern (nicht dargestellt).

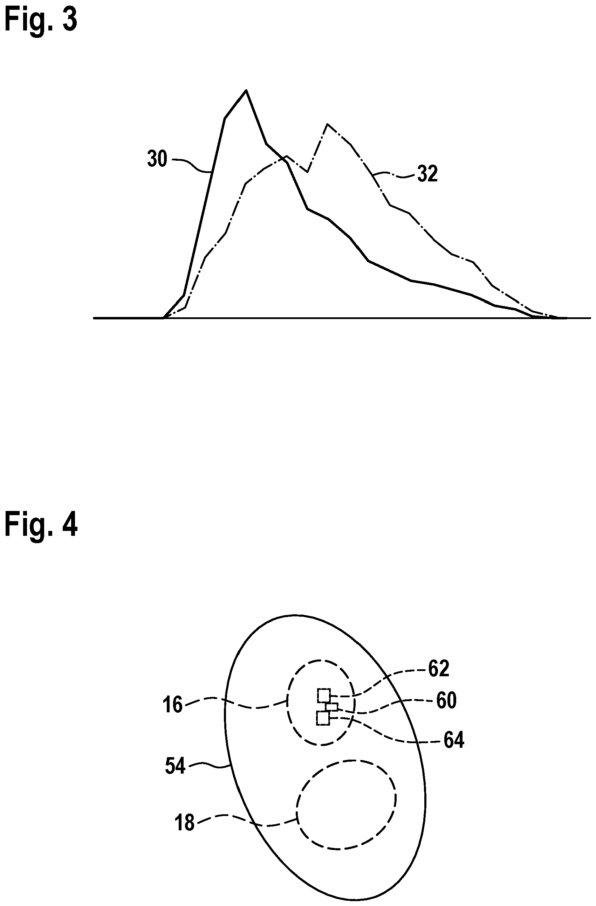

Die Analyseeinheit 22 ist im Lumineszenzmodus und im Hybridmodus dazu vorgesehen, ein erstes Lumineszenzsignal 30 des ersten Lumineszenzfarbstoffs 16 und ein zweites Lumineszenzsignal 32 des zweiten Lumineszenzfarbstoffs 18 in dem Abbild zu identifizieren. Die Analyseeinheit 22 kann dazu vorgesehen sein, gegebenenfalls mehr als zwei Lumineszenzfarbstoffe 16, 18 im Abbild zu identifizieren. Beispielhafte und von der Bildaufnahmeeinheit 20 in einem Betriebsmodus bereitgestellte Lumineszenzsignale 30, 32 in Form von Lumineszenzspektren sind in Fig. 3 gezeigt. Aufgrund der hyperspektralen Abbildung durch die Hyperspektralsensorik 66 liegt für jeden Bildpunkt des Abbilds eine entsprechende Spektralkurve, wie in Fig. 3 beispielhaft gezeigt, vor.

Die Analyseeinheit 22 ist dazu vorgesehen, die Lumineszenzsignale 30, 32 mit Referenzlumineszenzspektren verschiedener Lumineszenzfarbstoffe 16, 18 zu vergleichen. Dazu weist die Analyseeinheit 22 eine Kommunikationseinheit zur Kommunikation mit einer Internetdatenbank auf (nicht dargestellt), in welcher die Referenzlumineszenzspektren zugelassener Lumineszenzfarbstoffe 16, 18 hinterlegt sind. Alternativ oder zusätzlich kann die Analyseeinheit 22 über eine Speichereinheit verfügen, in welcher die Referenzlumineszenzspektren lokal abgespeichert sind.

Die Analyseeinheit 22 ist dazu vorgesehen, die Lumineszenzsignale 30, 32 auch bei sich teilweise überlappenden Lumineszenzspektren der Lumineszenzfarbstoffe 16, 18 zu identifizieren (vgl. Fig. 3). Hierzu analysiert die Analyseeinheit 22 für jeden Pixel der Hyperspektralsensorik 66 ein dort anliegendes Lumineszenzsignal und zerlegt dieses mittels gängiger Methoden in seine einzelnen Bestandteile. Diese vergleicht die Analyseeinheit 22 dann mit den Referenzlumineszenzspektren. Alternativ oder zusätzlich wäre denkbar, dass die Analyseeinheit 22 für jeden Pixel der Hyperspektralsensorik 66 ein dort anliegendes Lumineszenzsignal auf seine Maxima hin untersucht und diese Maxima mit den Maxima der Referenzlumineszenzspektren vergleicht.

Die Analyseeinheit 22 ist im Hybridmodus dazu vorgesehen, die Lumineszenzsignale 30, 32 auch bei einem gleichzeitig im Abbild vorhandenen Weißlichtbild 34 des Untersuchungsbereichs 14 zu identifizieren. Dabei macht sich die Analyseeinheit 22 die Tatsache zunutze, dass der Wellenlängenbereich des Weißlichtbilds 34 von dem Wellenlängenbereich der Lumineszenzsignale 30, 32 getrennt ist und dadurch eine unabhängige Analyse und Verarbeitung erfolgen kann.

Die Identifizierung der Lumineszenzfarbstoffe 16, 18 kann weiter verbessert werden, wenn die Analyseeinheit 22 dazu vorgesehen ist, zumindest eine Positionsinformation der zumindest zwei Lumineszenzfarbstoffe 16, 18 zu berücksichtigen. Wie in Fig. 4 gezeigt, kann die Analyseeinheit 22 dazu vorgesehen sein, bei einer Auswertung eines Lumineszenzsignals 30, 32 in einem Bereich 60 des Abbilds zumindest ein Lumineszenzsignal 30, 32 in einem Umgebungsbereich 62, 64 des Bereichs 60 zu berücksichtigen. Wenn in einem unmittelbar an den Bereich 60 angrenzenden Umgebungsbereich 62, 64 ein bestimmter der Lumineszenzfarbstoffe 16, 18 erkannt wurde, ist mit großer Wahrscheinlichkeit davon auszugehen, dass im Bereich 60 ebenfalls dieser Lumineszenzfarbstoff 16, 18 anzutreffen ist.

Die Analyseeinheit 22 kann dazu vorgesehen sein, bei einer Auswertung eines Lumineszenzsignals 30, 32 in einem Bereich 60 des Abbilds typische biologische Formen zu berücksichtigen. Beispielsweise sind Nervenbahnen oder Blutgefäße eher länglich, was bei der Unterscheidung und insbesondere Identifizierung, von Lumineszenzfarbstoffen 16, 18 durch die Analyseeinheit 22 berücksichtigt werden kann. Dabei kann die Analyseeinheit insbesondere berücksichtigen, wo sich diese in dem Gewebe 54 üblicherweise anreichern.

Im Weißlichtmodus gibt die Analyseeinheit 22 über die Anzeigeeinheit 44 ein reines Weißlichtbild, das heißt eine Reflektionsbild, aus dem Untersuchungsbereich 14 aus.

Im Lumineszenzmodus zeigt die Analyseeinheit 22 über die Anzeigeeinheit 44 ein reines Lumineszenzbild aus dem Untersuchungsbereich 14 an, wobei Lumineszenzsignale 30, 32 unterschiedlich farbig dargestellt sein können.

Im Hybridmodus ist die Analyseeinheit 22 dazu vorgesehen, die Lumineszenzsignale 30, 32 über die Anzeigeeinheit 44 gemeinsam als verschiedenfarbige Overlays 36, 38 über dem Weißlichtbild 34 darzustellen (vgl. Fig. 1).

Ein Bediener der Bildgebungsvorrichtung 10 kann insbesondere während einer Untersuchung und/oder eines Eingriffs zwischen den verschiedenen Betriebsmodi umschalten, um eine optimale Darstellung mit einem für ihn optimalen Informationsgehalt zu erzielen.

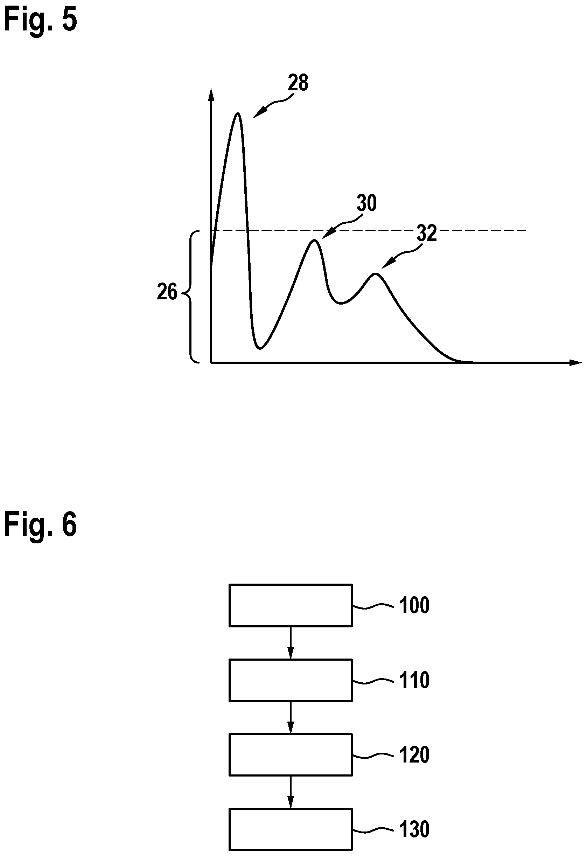

Die Analyseeinheit 22 ist dazu vorgesehen ist, zumindest einen Belichtungsparameter der Hyperspektralsensorik 66 so einzustellen, dass zumindest ein Lumineszenzsignal 30, 32 eines Lumineszenzfarbstoffs 16, 18 wesentlich ausgesteuert ist. Dies ist in Fig. 5 beispielhaft dargestellt. Der Belichtungsparameter wird für auf eine Bildaufnahme folgende Bildaufnahmen der Hyperspektralsensorik 66 so eingestellt, dass eines der Lumineszenzsignale 30 einen Detektionsbereich 26 im Wesentlichen voll aussteuert, wohingegen die übrigen Lumineszenzsignale 32 eine geringere Aussteuerung aufweisen. Ein Reflektionssignal 28 des Anregungslichts ist dann übersteuert, was jedoch keine Rolle spielt, da dieses nicht für eine Darstellung auf der Anzeigeeinheit 44 verwendet wird.

Fig. 6 zeigt ein Schaubild eines Verfahrens zum Betrieb der Bildgebungsvorrichtung 10. In dem Lumineszenzmodus oder dem Hybridmodus wird in einem Schritt 100 der Untersuchungsbereich 14 bestrahlt. Dabei werden gleichzeitig der erste Lumineszenzfarbstoff 16 und der zweite Lumineszenzfarbstoff 18 angeregt. In einem Schritt 110 wird ein hyperspektrales Abbild des Untersuchungsbereichs 14 aufgenommen. In einem Schritt 120 wird das Abbild analysiert und die Lumineszenzfarbstoffe 16, 18 werden anhand ihrer Lumineszenzspektren identifiziert. Dies erfolgt durch Vergleich mit Referenzlumineszenzspektren von Lumineszenzfarbstoffen. In einem Schritt 130 wird das Lumineszenzbild ausgegeben.

### Bezugszeichenliste

- 10: Bildgebungsvorrichtung
- 12: Beleuchtungsvorrichtung
- 14: Untersuchungsbereich
- 16: erster Lumineszenzfarbstoff
- 18: zweiter Lumineszenzfarbstoff
- 20: Bildaufnahmeeinheit
- 22: Analyseeinheit
- 24: Objektivlinse
- 26: Detektionsbereich
- 28: Reflektionssignal
- 30: erstes Lumineszenzsignal
- 32: zweites Lumineszenzsignal
- 34: Weißlichtbild
- 36: erstes Overlay
- 38: zweites Overlay
- 40: Zeilensensor
- 42: Matrixdetektor
- 44: Anzeigeeinheit
- 46: Bildgebungsgerät
- 48: optische Schnittstelle
- 50: Beleuchtungsquelle
- 52: Beleuchtungsquelle
- 54: Gewebe
- 56: PGP-Anordnung
- 58: Schlitzblende
- 60: Bereich
- 62: Umgebungsbereich
- 64: Umgebungsbereich
- 66: Hyperspektralsensorik
- 68: Lichtleiter
- 70: Kabel
- 72: Breitbandoptik
- 73: erste Raumkoordinate
- 74: zweite Raumkoordinate
- 100: Schritt
- 110: Schritt
- 120: Schritt
- 130: Schritt

## Patentansprüche

1. Bildgebungsvorrichtung (10), insbesondere endoskopische, exoskopische und/oder mikroskopische Bildgebungsvorrichtung, umfassend:
- eine Beleuchtungsvorrichtung (12), welche zur Beleuchtung eines Untersuchungsbereichs (14) vorgesehen ist,
- eine Bildaufnahmeeinheit (20), welche eine Hyperspektralsensorik (66) zur Aufnahme zumindest eines hyperspektralen Abbilds des Untersuchungsbereichs (14) aufweist, und
- eine Analyseeinheit (22) zur Analyse des Abbilds,
**dadurch gekennzeichnet, dass**
die Beleuchtungsvorrichtung (12) zur gleichzeitigen Anregung eines ersten Lumineszenzfarbstoffs (16) und zumindest eines zweiten Lumineszenzfarbstoffs (18) vorgesehen ist.

2. Bildgebungsvorrichtung (10) nach Anspruch 1,
**dadurch gekennzeichnet, dass**
die Bildaufnahmeeinheit (20) eine Breitbandoptik (72) aufweist, welche an einem Eingang der Hyperspektralsensorik (66) angeordnet ist.

3. Bildgebungsvorrichtung (10) nach einem der vorhergehenden Ansprüche, **dadurch gekennzeichnet, dass**
die Hyperspektralsensorik (66) eine Scaneinheit zu einem Abscannen zumindest einer Raumrichtung des Abbilds aufweist.

4. Bildgebungsvorrichtung (10) nach einem der vorhergehenden Ansprüche, **dadurch gekennzeichnet, dass**
die Beleuchtungsvorrichtung (12) dazu vorgesehen ist, Weißlicht zur Beleuchtung des Untersuchungsbereichs (14) gleichzeitig zu einem Anregungslicht zur Anregung der zumindest zwei Lumineszenzfarbstoffe (16, 18) bereitzustellen.

5. Bildgebungsvorrichtung (10) nach einem der vorhergehenden Ansprüche, **dadurch gekennzeichnet, dass**
die Beleuchtungsvorrichtung (12) eine Beleuchtungsquelle (50) aufweist, welche zur gleichzeitigen Anregung der zumindest zwei Lumineszenzfarbstoffe (16, 18) vorgesehen ist.

6. Bildgebungsvorrichtung (10) nach dem Oberbegriff von Anspruch 1 und insbesondere nach einem der vorhergehenden Ansprüche,
**dadurch gekennzeichnet, dass**
die Analyseeinheit (22) dazu vorgesehen ist, ein erstes Lumineszenzsignal (30) eines ersten Lumineszenzfarbstoffs (16) und zumindest ein zweites Lumineszenzsignal (32) zumindest eines zweiten Lumineszenzfarbstoffs (18) in dem Abbild zu unterscheiden, vorzugsweise zu identifizieren.

7. Bildgebungsvorrichtung (10) nach Anspruch 6,
**dadurch gekennzeichnet, dass**
die Analyseeinheit (22) dazu vorgesehen ist, die zumindest zwei Lumineszenzsignale (30, 32) mit Referenzlumineszenzspektren verschiedener Lumineszenzfarbstoffe (16, 18) zu vergleichen.

8. Bildgebungsvorrichtung (10) nach Anspruch 6 oder 7,
**dadurch gekennzeichnet, dass**
die Analyseeinheit (22) dazu vorgesehen ist, die zumindest zwei Lumineszenzsignale (30, 32) auch bei sich teilweise überlappenden Lumineszenzspektren der zumindest zwei Lumineszenzfarbstoffe (16, 18) zu unterscheiden, vorzugsweise zu identifizieren.

9. Bildgebungsvorrichtung (10) nach einem der Ansprüche 6 bis 8,
**dadurch gekennzeichnet, dass**
die Analyseeinheit (22) dazu vorgesehen ist, die zumindest zwei Lumineszenzsignale (30, 32) auch bei einem gleichzeitig im Abbild vorhandenen Weißlichtbild (34) des Untersuchungsbereichs (14) zu unterscheiden, vorzugsweise zu identifizieren.

10. Bildgebungsvorrichtung (10) nach Anspruch 9,
**gekennzeichnet durch**
eine Anzeigeeinheit (44) zur Darstellung einer Ansicht wenigstens eines Teils des Untersuchungsbereichs (14),
wobei die Analyseeinheit (22) dazu vorgesehen ist, die zumindest zwei Lumineszenzsignale (30, 32) über die Anzeigeeinheit (44) in der Ansicht gemeinsam als verschiedenartig, vorzugsweise verschiedenfarbig, gekennzeichnete Overlays (36, 38) über dem Weißlichtbild (34) darzustellen.

11. Bildgebungsvorrichtung (10) nach einem der vorhergehenden Ansprüche, **dadurch gekennzeichnet, dass**
die Bildaufnahmeeinheit (20) und/oder die Analyseeinheit (22) dazu vorgesehen ist, zumindest einen Belichtungsparameter der Hyperspektralsensorik (66) so einzustellen, dass zumindest ein Lumineszenzsignal (30, 32) eines Lumineszenzfarbstoffs (16, 18) wesentlich ausgesteuert ist.

12. Bildgebungsvorrichtung (10) nach einem der vorhergehenden Ansprüche, **dadurch gekennzeichnet, dass**
die Analyseeinheit (22) dazu vorgesehen ist, bei einer Auswertung eines Lumineszenzsignals (30, 32) in einem Bereich (60) des Abbilds zumindest ein Lumineszenzsignal (30, 32) in einem Umgebungsbereich (62, 64) des Bereichs (60) zu berücksichtigen.

13. Bildgebungsvorrichtung (10) nach einem der vorhergehenden Ansprüche, **dadurch gekennzeichnet, dass**
die Analyseeinheit (22) dazu vorgesehen ist, bei einer Auswertung eines Lumineszenzsignals (30, 32) in einem Bereich (60) des Abbilds typische biologische Formen zu berücksichtigen.

14. Bildgebungsvorrichtung (10) nach einem der vorhergehenden Ansprüche, **dadurch gekennzeichnet, dass**
die Beleuchtungsvorrichtung (12) dazu vorgesehen ist, zumindest eine Information hinsichtlich eines zur Beleuchtung des Untersuchungsbereichs (14) verwendeten Beleuchtungsspektrums an die Bildaufnahmeeinheit (20) und/oder an die Analyseeinheit (22) zu übermitteln.

15. Verfahren zum Betrieb einer Bildgebungsvorrichtung (10), insbesondere einer endoskopischen, exoskopischen und/oder mikroskopischen Bildgebungsvorrichtung, insbesondere nach einem der vorhergehenden Ansprüche,
wobei ein Untersuchungsbereich (14) bestrahlt wird und dabei gleichzeitig ein erster Lumineszenzfarbstoff (16) und zumindest ein zweiter Lumineszenzfarbstoff (18) angeregt werden und
wobei ein hyperspektrales Abbild des Untersuchungsbereichs (14) aufgenommen wird.
